# EUROPEAN PATENT APPLICATION

(11) **EP 4 684 795 A1**
(43) Date of publication of application: **28.01.2026**
(21) Application number: 24773824.8
(22) Date of filing: 06.02.2024
(51) Int. Cl.: A61K 38/12, A61K 38/15, A61P 27/02

(54) **USE OF BEAUVERICIN IN PREPARATION OF DRUG FOR INHIBITING ANGIOGENESIS**

(30) Priority: 20.03.2023 US 202363453225 P
(71) Applicant: Zih Yuan Tang Biotechnology Co., Ltd., Taipei City 10677 Taiwan (TW)
(72) Inventor: SU, Ming-Jai, Taipei City, Taiwan 10677 (CN); LEE, Shoei-Sheng, Taipei City, Taiwan 10677 (CN); TSAI, Feng-Chiao, Taipei City, Taiwan 10677 (CN); CHEN, Hou-Jen, Taipei City, Taiwan 10677 (CN); HSU, Chao-Min, Taipei City, Taiwan 10677 (CN)
(74) Representative: Straus, Alexander
(86) International application number: PCT/CN2024/076466
(87) International publication number: WO 2024/193256

(57) **Abstract**

The present invention provides a use of beauvericin in manufacturing a medicament for inhibiting angiogenesis, in which the therapeutically effective concentration of beauvericin ranges from 0.1 µM to 5 µM; which can be used for preparation of a medicament for preventing or treating a retinal disorder, or a pharmaceutical composition or combination with an anti-cancer drug for treating a cancer.

## Description

### FIELD OF THE INVENTION

This invention provides a use of beauvericin in manufacturing a medicament for inhibiting angiogenesis, which can be used to develop a medicament for treating or preventing a retinal disorder, or a pharmaceutical composition or combination for treating a cancer.

### BACKGROUND OF THE INVENTION

Cancer is caused by the abnormal hypertrophy and proliferation of cells, which migrates and invades other tissues through the circulatory system or lymphatic circulation system in a subject, resulting in organ dysfunction and failure. It has been demonstrated that there is a significant correlation between cancer cells and angiogenesis, a process in which pre-existing vessels grow by sprouting another blood vessel. Tumor cells require a large amount of nutrients and oxygen to grow and spread, which are supplied through blood vessels. Tumors usually promote angiogenesis of the surrounding tissues to form more blood vessel networks to meet their growth and metabolic needs. Therefore, inhibition of angiogenesis is effective in controlling the development of cancer to a considerable extent.

Angiogenesis is also related to the occurrence of relevant eye diseases. In situations of poor blood circulation in the eyes, it often leads to hypoxia in retinal tissues and the secretion of vascular endothelial growth factor (VEGF). While VEGF is supposed to induce compensatory angiogenesis to counteract the hypoxic environment, in most pathological conditions, these newly formed blood vessels are small and dense. They not only fail to increase blood flow effectively but also contribute to increased permeability and leakage of the vessel walls. The accumulated fluid further hinders blood flow, creating a vicious cycle.

However, it is still desirable to develop a medicament for inhibiting angiogenesis, which can be used for prevention or treatment of a retinal disorder, or treatment of a cancer.

### SUMMARY OF THE INVENTION

Accordingly, the present invention provides a use of a compound in manufacturing a medicament for inhibiting angiogenesis, wherein the compound is beauvericin having the following structure, as known as GYT-088 hereinafter, at a therapeutically effective concentration ranging from 0.1 µM to 5 µM:

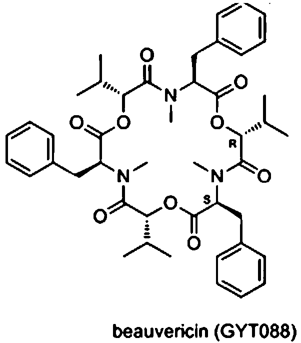

In one embodiment of the present invention, the medicament for inhibiting angiogenesis can be used for development of a medicament for treating or preventing a retinal disorder.

In one example of the present invention, the retinal disorder is selected from the group consisting of age-related macular degeneration (AMD), glaucoma, and diabetic retinopathy.

According to the example of the present invention, for humans, the therapeutically effective concentration of beauvericin is preferably 0.5 µM to 5 µM.

According to the example of the present invention, the therapeutically effective dose for treating or preventing a retinal disorder ranges from 2.0 to 5.5 µg per eye.

In the other aspect, the present invention provides a pharmaceutical composition for inhibiting angiogenesis comprising beauvericin at a therapeutically effective concentration ranging from 0.1 µM to 5 µM, and a pharmaceutically acceptable carrier.

According to the example of the present invention, the therapeutically effective concentration of beauvericin is preferably 0.5 µM to 5 µM.

In one example of the present invention, the pharmaceutical composition can be used for development of a pharmaceutical or combination for treating a cancer in association with an anti-cancer drug.

It is to be understood that both the foregoing general description and the following detailed description are exemplary and explanatory only and are not restrictive of the invention.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred.

In the drawings:

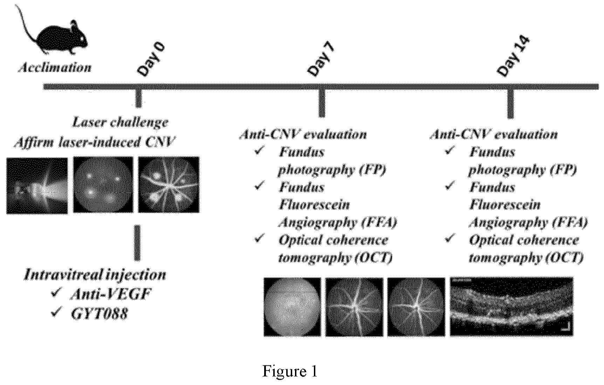

Figure 1 provides a schematic diagram showing the timeline and flow of the experiment process.

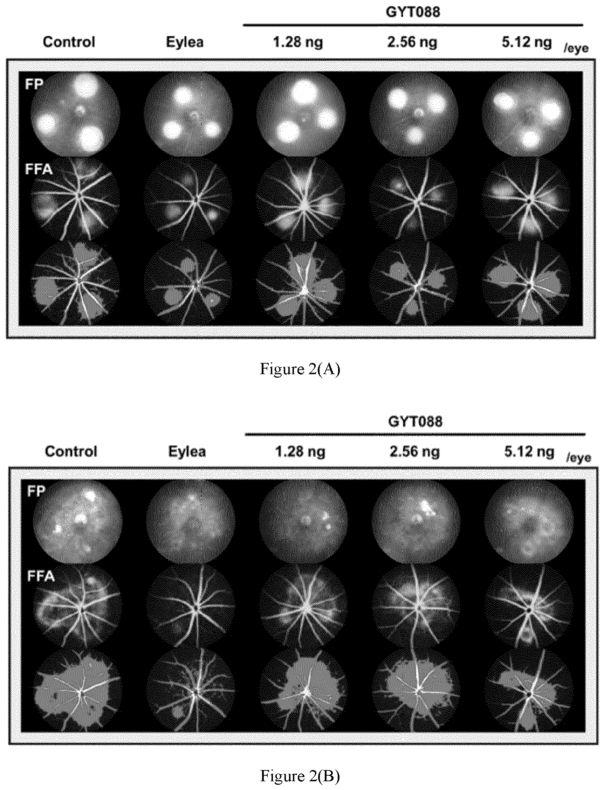

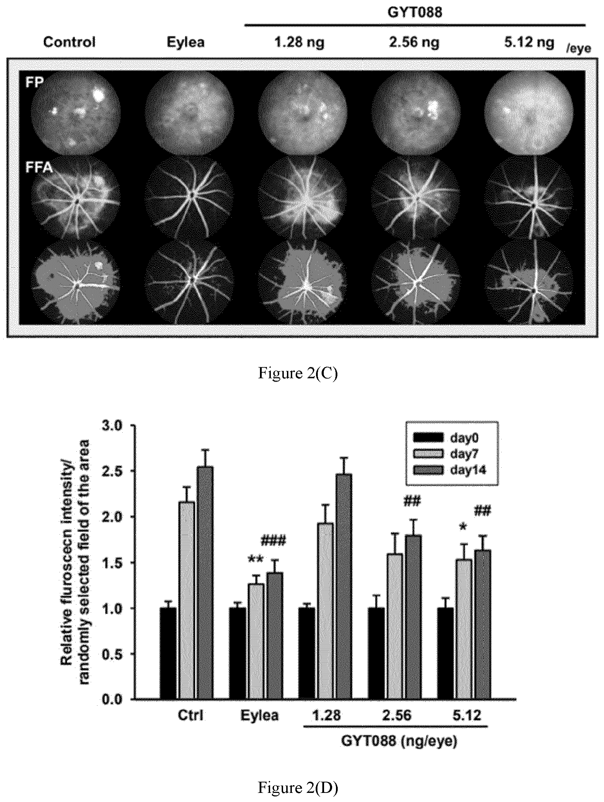

Figure 2 provides the image and analysis data showing the results of the fundus photography (FP) and fundus fluorescein angiography (FFA); wherein Figure 2(A) shows the result at Day 0 after laser challenge; Figure 2(B) shows the result at Day 7 after laser challenge; Figure 2(C) shows the result at Day 14 after laser challenge; Figure 2(D) provides the quantified values of the fundus luciferin angiography detection (*p<0.05 means a significant difference between the high-dose GYT088 treatment group (5.12 ng/eye) and other groups ar Day 7; **p<0.01 means a significant difference between the Eylea treatment group and other groups at Day 7; ^{##}p<0.01 means a significant difference between the mid-dose, high-dose GYT088 treatment groups (2.56, 5.12 ng/eye) and other groups at Day 14; ^{###}p<0.01 means a significant difference between the Eylea treatment group and other groups at Day 14).

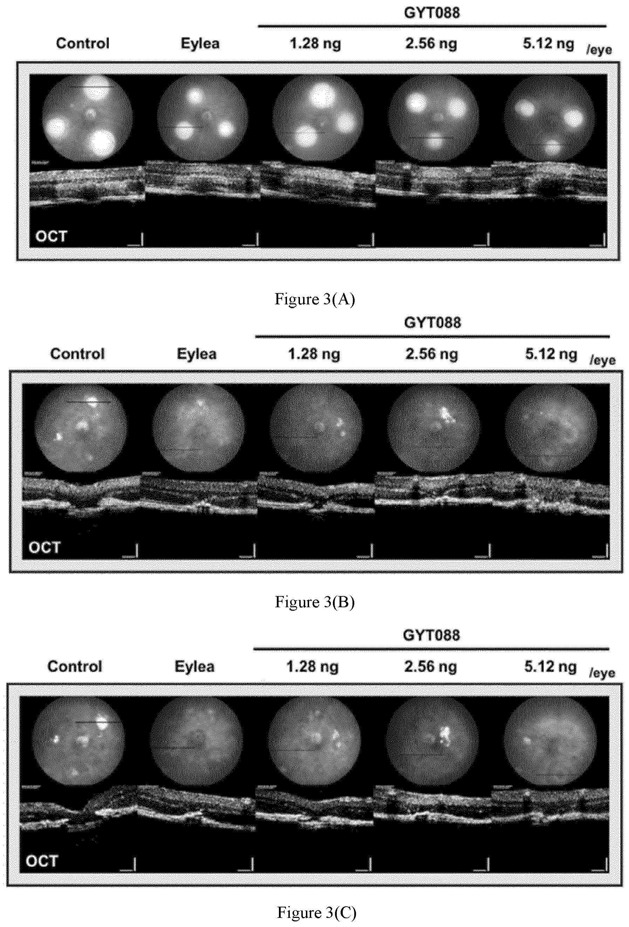

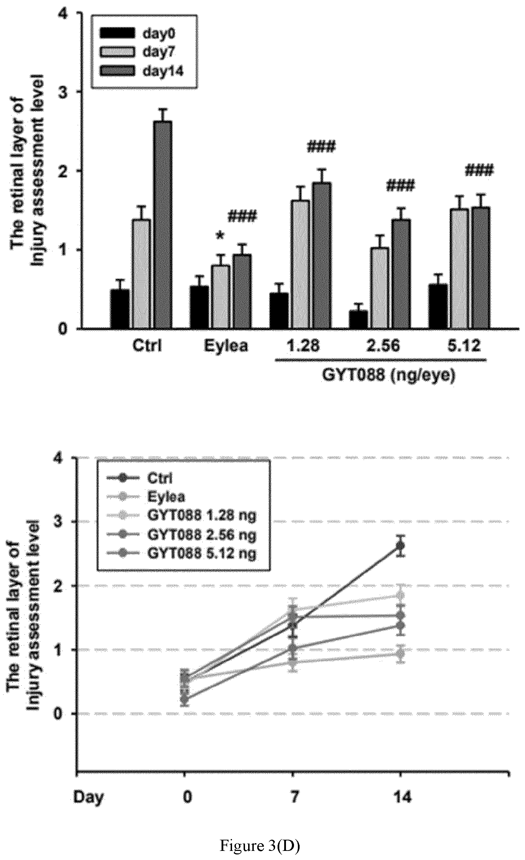

Figure 3 provides the image and analysis data showing the observations of the laser injury at retinal layer by spectral-domain optical coherence tomography (SD-OCT); wherein Figure 3(A) shows the result at Day 0 after laser challenge; Figure 3(B) shows the result at Day 7 after laser challenge; Figure 3(C) shows the result at Day 14 after laser challenge; and Figure 3(D) provides the quantified values of the spectral-domain optical coherence tomography (*p<0.05 means a significant difference between the Eylea treatment group and other groups at Day 7; ^{###}p<0.01 means a significant difference between Eylea, low-dose, mid-dose, high-dose GYT088 treatment groups (1.28, 2.56. 5.12 ng/eye) and other groups at Day 14).

### DETAILED DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereto known to those skilled in the art.

The term "subject" as used herein refers to any human or nonhuman animal. The term "nonhuman animal" includes, but is not limited to, vertebrates such as nonhuman primates, sheep, dogs, cats, rabbits, ferrets, rodents such as mice, rats and guinea pigs, avian species such as chickens, amphibians, and reptiles. In more preferred embodiments, the subject is a human. The terms, "subject," "patient" and "individual" are used interchangeably herein.

The term "angiogenesis" as used herein refers to the process of new blood vessel formation occurs when vascular endothelial growth factor (VEGF) promotes the endothelial growth or migration of vascular cells for tissue growth, healing or repair. This physiological process is essential for normal development and maintenance of tissue physiology, however, when angiogenesis is out of balance, it can also lead to disease, particularly in diseases such as cancer and chronic inflammation.

The term "cancer" as used herein refers to a disease that develops when the abnormal cells grow and divide uncontrollably. These abnormal cells are called tumors, and cancer is usually referred to as a malignant tumor that divides and invades other normal cells and tissues through the body's circulation, causing dysfunction or organ failure.

The term "a retinal disorder" or "retinopathy" as used herein refers to various diseases that affect the retinal tissue of the eye. Since the retina is the tissue inside the eye that receives light and converts it into neural signals, then be sent to the brain for visual perception. Damage or disease to the retina would lead to vision impairment or loss. Common retinal disorders include age-related macular degeneration, diabetic retinopathy, glaucomatous optic neuropathy, retinal detachment and Retinal Vascular Diseases.

As used herein, the term "therapeutically effective concentration" or "therapeutically effective dose" refers to a concentration or amount, as compared to a corresponding patient who has not received such concentration or amount, results in an effect in treatment, healing, prevention, or amelioration of a disease, disorder, or side effect, or a decrease in the rate of advancement of a disease or disorder. The term also includes within its scope amounts effective to enhance normal physiological function.

The term "beauvericin" as used herein refers to a compound produced by fungi of the genus "Beauveria" as a secondary metabolite. It belongs to a group of compounds known as cyclic peptides and has a specialized structure below:

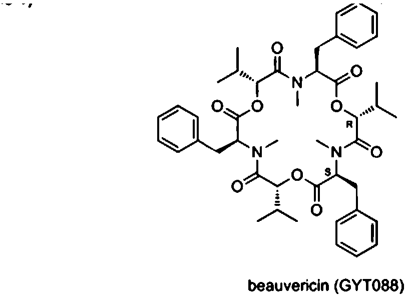

The structural features of beauvericin include an alternating arrangement of three D-hydroxyvaleric acids and three N-methyl-phenylalanines. Beauvericin has a wide range of biological activities, including anti-microbial, insecticidal, and anti-cancer activities against a variety of tumor cell lines.

In the present invention, it is discovered that beauvericin at a given low amount exhibits an efficacy in inhibition of angiogenesis, which can be used to develop a medicament for preventing or treating a retinal disorder, or a pharmaceutical composition or combination for treating a cancer in association with an anti-cancer drug.

According to the invention, the therapeutically effective amount (dose) for a human is calculated as 2.56 µg to 5.12 µg per eye based on the experiments in mice. Accordingly, it can be evaluated that the concentration of beauvericin effective to inhibit angiogenesis is in a range of 0.1 µM to 5 µM, preferably 0.5 µM to 5 µM.

For therapeutic use, a therapeutically effective concentration of the compound is formulated into a pharmaceutical composition for administration. Therefore, the present invention further provides a pharmaceutical composition comprising beauvericin at a therapeutically effective concentration ranging from 0.1 µM to 5 µM, and a pharmaceutically acceptable carrier.

The term "pharmaceutically acceptable carrier" used herein refers to a carrier(s), diluent(s) or excipient(s) that is acceptable, in the sense of being compatible with the other ingredients of the formulation and not deleterious to the subject to be administered with the pharmaceutical composition. Any carrier, diluent or excipient commonly known or used in the field may be used in the invention, depending to the requirements of the pharmaceutical formulation.

According to the invention, the pharmaceutical composition may be adapted for administration by any appropriate route, including but not limited to oral, rectal, nasal, topical, vaginal, or parenteral route. In one particular example of the invention, the pharmaceutical composition is formulated for oral administration. Such formulations may be prepared by any method known in the art of pharmacy.

The present invention is further illustrated by the following examples, which are provided for the purpose of demonstration rather than limitation.

### Example

### Experiment of beauvericin improving retinopathy

### 1. Materials

Beauvericin (BEA), named GYT088 in this trail, was supplied by Shiyuantang Biotechnology Co. 5 mg of BEA (molecular weight 783.96) was dissolved in DMSO at a stock concentration of 50 mM, and then diluted to 100 µM with saline. Before injection, the sample was diluted to 1.6, 3.2, and 6.4 µM in saline, and then administered to the low, medium, and high-dose groups, which were equivalent to 1.28, 2.56, and 5.12 ng/eye, respectively, based on a vitreous volume of 4 µl in mice (wherein the injection volume is 1 µl); converted to the human doses of 1.28, 2.56, 5.12 µg/eye as the low, medium and high dose levels, respectively, based on a vitreous volume of 4-4.5 ml in human.

### 2. Design of Experiment

Male C57BL/6 mice from BioLASCO Taiwan CO., Ltd. at 7-8 weeks of age, with an average body weight of 17-20 g, were used in this study. The mice were housed in the Animal Room of the National Taipei Medical University for more than 5 days before the start of the experiment. The incubation temperature was set at 23±1°C, the humidity was set at 39~43%, and the automated light control system maintained 12 hours of light and 12 hours of darkness. The feed was Rodent diet 5001 (Purina, MO, USA) and drinking water was supplied indefinitely.

After the adaptation period, the retina was cauterized with laser light (day 0), and the degree of retinal damage was confirmed by fundus photography (FP), fluorescein fundus angiography (FFA), and frequency-domain optical co-modulation tomography (SD-OCT) on the same day (Figure 1). The mice were randomly divided into five groups and given vitreous injections of the corresponding drugs, including (1) control group, (2) positive control group: Eylea (40 µg/eye/ µL), (3-5) GYT088 treatment groups at low-dose (1.28 ng/eye), medium-dose (2.56 ng/eye), and high-dose (5.16 ng/eye). Five mice in each group were clinically observed daily, and retinal injury repair was examined at Day 7 and Day 14.

### 3. Methods

### 3.1 Laser-induced choroidal neovascularization

While this specification contains many specifics, these should not be construed as limitations on the scope of the invention or of what may be claimed, but rather as descriptions of features specific to particular embodiments or examples of the invention. Certain features that are described in this specification in the context of separate embodiments or examples can also be implemented in combination in a single embodiment.

### 3.2 Fundus fluorescein angiography (FFA)

Mice were deeply anesthetized and pupils were dilated. A Micron III retinal imaging microscope (Phoenix, San Ramon, CA, USA) was used to adjust the focal length, and the central socket of the mice was placed in the center of the fundus lens to capture fundus photography (FP), and then 10% sodium fluorescein solution was injected intraperitoneally, and retinal angiography was performed with a 520 nm filter to capture continuous images for analysis. After the administration of drugs, the FP, FFA, and SD-OCT results were observed once a week at Day 0 and recorded until Day 14. Sharp images were selected for analysis and imported into 16-pixel format using ImageJ, and the software automatically quantified the pixel value and area of the fundus region by removing the background values. Due to differences in individual retinal structures, post-laser wound healing varies and overexposure of signals in the area may occur. If fluorescence leakage occurs outside of the location of direct laser damage, it is considered to be a phenomenon of fundus neovascularization. Therefore, the quantification was performed by randomly selecting three fixed areas of the fluorescence leakage area that were not laser-damaged areas.

### 3.3 Spectral-domain optical coherence tomography (SD-OCT) and analysis

The OCT (Phoenix Research Laboratories, San Ramon, CA) imaging system used in this experiment is based on the principle of frequency-domain optical homodyne tomography. The computerized tomography (CT) scan was divided into three layers: the first layer was from the nerve fiber layer (NFL) to the inner nuclear layer (INL), the second layer was from the INL to the inner segment/outer segment (IS/OS), and the third layer was from the IS/OS to the retinal pigment epithelium (RPE). The damage was scored according to the retinal sublayer structure in a double-blind trail, and the mean value of the scores was taken as the index value of the damage after each score was made by 3 persons. The scoring criteria were as follows:

**Table 1. The damage score of the retinal sublayer structure**

| Score | Retinal sublayer structure (Retinal layer observation) |
|---|---|
| 0 | No inflammation, No hyper-reflective foci (HRF) |
| 1 | HRF in the outer retina without affecting the INL |
| 2 | HRF in the inner retina |
| 3 | HREF in the inner retina, damage to the retinal sub-structure |
| 4 | Hydrocolloid Cyst |

### 3.4 Statistical analysis

All data were presented as mean ± standard deviation (S.E.M.) and statistically analyzed by one-way ANOVA (Turkey test). p<0.05 means significant differences.

### 4. Results

### 4.1 Image analysis of FFA

FFA images were analyzed by ImageJ image analysis to quantify the area of fluorescence leakage. Since there was no significant leakage at Day 0, the fluorescence obtained was mainly from new blood vessels. 3 laser-induced wounds were observed in the fundus from each group of mice on day 0, and the relative average fluorescence leakage ratios at Days 0, 7, and 14 after laser injury were quantified (Figure 2). The angiogenesis indexes in GYT088 treatment groups at Day 7 from the low to high-dose groups were 1.92±0.21, 1.59±0.22, and 1.53±0.17 times respectively, higher than that on day 0, wherein the inhibition of angiogenesis in the high-dose treatment group had a statistical difference (p<0.05). The angiogenesis indexes at Day 14 in the GYT088 treatment group from the low to high-dose groups were 2.46±0.18, 1.79±0.17, and 1.63±0.16 times respectively compared with Day 0, and the inhibition of angiogenesis in the medium and high-dose treatment groups showed a statistical difference (p<0.01). Since the inhibition of GYT088 showed a dose- and time-dependent trend, it was considered that vitreous injection of GYT088 (2.56 and 5.12 ng/eye) had the efficacy of inhibiting laser-induced angiogenesis. Converted human dose ranges were 25.6 and 51.2 ng/eye.

### 4.2 Analysis of retinal sublayer structure

OCT images were used to observe the structural integrity of the retinal sublayer and some substructural changes, for example, some hyperreflective nodes may be caused by the aggregation of immune cells or pigment epithelial cell migration. Retinal cysts were also observed on OCT images, in order to compare the degree of damage to the retina in each group the groups from funduscopic photographs, a scale of 0-4 was assigned to each group of funduscopic photographs according to the severity of the disease and the results of 3 groups of blinded trails were quantified and statistically analyzed (Figure 3). Results showed that the damage scores of each group at Day 7 ranged from 0.80 to 1.62, and the damage score of the Eylea group at Day 7 (0.80 ± 0.13) was significantly lower than control group (1.38 ± 0.17), which showed that the Eylea could protect the retinal sub-structure. On day 14, the damage scores of each group ranged from 0.93 to 2.62, and the damage score of high-dose GYT088 (5.12 ng/eye) treatment group was also significantly lower than control group, which showed that GYT088 could maintain the sublayer of the retina and reduce the degree of cell aggregation.

While this specification contains many specifics, these should not be construed as limitations on the scope of the invention or of what may be claimed, but rather as descriptions of features specific to particular embodiments or examples of the invention. Certain features that are described in this specification in the context of separate embodiments or examples can also be implemented in combination in a single embodiment.

## Claims

1. A use of a compound for manufacturing a medicament for inhibiting angiogenesis, in which said compound is beauvericin having the structure of the formula below at a therapeutically effective concentration ranging from 0.1 µM to 5 µM:

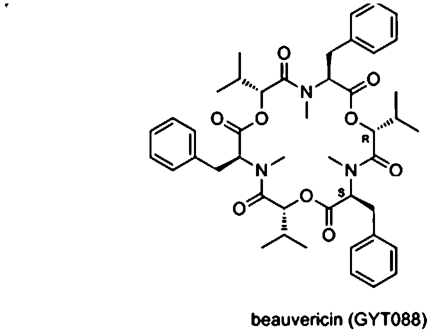

2. The use of claim 1, wherein the therapeutically effective concentration of beauvericin is 0.5 µM to 5 µM.

3. The use of claim 1, wherein the medicament for inhibiting angiogenesis is used to develop a pharmaceutical composition for prevention or treatment of a retinal disorder.

4. The use of claim 3, wherein the retinal disorder is selected from the group consisting of age-related macular degeneration (AMD), glaucoma, and diabetic retinopathy.

5. A pharmaceutical composition for inhibiting angiogenesis, which comprises beauvericin at a therapeutically effective concentration ranging from 0.1 µM to 5 µM, and a pharmaceutically acceptable carrier.

6. The pharmaceutical composition of claim 5, wherein the therapeutically effective concentration is 0.5 µM to 5 µM.

7. The pharmaceutical composition of claim 5, which is effective in treatment or prevention of a retinal disorder.

8. The pharmaceutical composition of claim 5, which is effective in treatment of a cancer in association with an anti-cancer drug.
